Europäisches Patentamt

European Patent Office

Office européen des brevets

(11) Veröffentlichungsnummer: **0 265 712**

**A1**

(12) # EUROPÄISCHE PATENTANMELDUNG

(21) Anmeldenummer: **87114447.3**

(22) Anmeldetag: **03.10.87**

(51) Int. Cl.4: **A61F 2/34**

(30) Priorität: **16.10.86 CH 4141/86**
**07.11.86 CH 4467/86**

(43) Veröffentlichungstag der Anmeldung:
**04.05.88 Patentblatt 88/18**

(84) Benannte Vertragsstaaten:
**CH DE FR GB IT LI NL**

(71) Anmelder: **Protek AG**
**Stadtbachstrasse 64**
**CH-3012 Bern(CH)**

(72) Erfinder: **Müller, Maurice E.,Prof. Dr. med.**
**Melchenbühlweg 9**
**CH-3006 Bern(CH)**

(74) Vertreter: **Lusuardi, Werther G.**
**Dr. Lusuardi AG Stockerstrasse 8**
**CH-8002 Zürich(CH)**

(54) **Acetabularteil für eine Hüfttotalprothese.**

(57) Das Acetabularteil für eine Hüfttotalprothese besteht aus einer metallischen Verankerungsschale (1), deren dem Beckenknochen zugewandte konvexe Fläche im wesentlichen die Form einer Kugelkalotte hat, mit einem in die Verankerungsschale (1) einsetzbaren Pfanneneinsatz (2), dessen äussere konvexe Form so auf die innere konkave Form der Verankerungsschale (1) abgestimmt ist, dass sie ohne Verwendung von Knochenzement in der Verankerungsschale (1) verdrehsicher fixiert werden kann und dessen innere konkave Form in an sich bekannter Weise die reibungsarme und ungestörte Bewegung eines künstlichen Femurkugelkopfes ermöglicht. Die Verankerungsschale (1) weist mehrere längs Meridianen der Kugelkalotte angeordnete durchgehende Schlitze (9) und mehrere zwischen der Aequatorlinie und dem Pol der Kugelkalotte liegende Bohrungen (6) zur zementlosen Primärverankerung im Beckenknochen mittels Verankerungsschrauben (3) auf.

Die wesentlichen Vorteile der kombinierten Anordnung von Schlitzen (9) und Bohrungen (6) gemäss der Erfindung liegen darin, dass unmittelbar nach der Implantation des Acetabularteils eine genügende Primärfixation erreicht wird, während durch die Möglichkeit der Einfügung von Spongiosamaterial in den Bereich der Schlitze (9) in erster Linie eine Rotationssicherung erreicht wird.

Fig. 2

## Acetabularteil für eine Hüfttotalprothese

Die Erfindung betrifft ein Acetabularteil für eine Hüfttotalprothese, bestehend aus einer metallischen Verankerungsschale, deren dem Beckenknochen zugewandte konvexe Fläche im wesentlichen die Form einer Kugelkalotte hat, mit Bohrungen zur zementlosen Primärverankerung im Beckenknochen mittels Verankerungsschrauben und einem in die Verankerungsschale einsetzbaren Pfanneneinsatz, dessen äussere konvexe Form so auf die innere konkave Form der Verankerungsschale abgestimmt ist, dass sie ohne die Verwendung von Knochenzement in der Verankerungsschale verdrehsicher fixiert werden kann und dessen innere konkave Form in an sich bekannter Weise eine reibungsarme und ungestörte Bewegung eines künstlichen Femurkopfes ermöglicht.

Gelenkpfannen als Gegenlager für den künstlichen Femurkopf werden bevorzugt aus hochpolymerem Polyaethylen hergestellt. Diese Kunststoffpfannen werden oft direkt in das entsprechend ausgefräste Acetabulum implantiert. Nach der heute vorherrschenden Meinung ist jedoch eine Knochen-Metall-Polyaethylen-Konfiguration der Knochen-Polyaethylen-Konfiguration vorzuziehen, wie z.B. von Mark B. Coventry in the Year book of Orthopaedics, Chicago, Year Book Medical Publishers Inc. 1985, S. 175, dargelegt.

Zur Verwirklichung der künstlichen Hüftpfanne in der heute bevorzugten Knochen-Metall-Polyaethylen-Konfiguration ist z.B. aus der europäischen Patentanmeldung 0142759 ein Metallring mit Gewinde bekannt, der in das Acetabulum eingedreht wird und in welchen eine Polyaethylenpfanne eingeklemmt wird.

Es sind auch Polyaethylen-Hüftgelenkpfannen bekannt, deren Rand teilweise überhöht ist, d.h. eine teilweise erhöhte Schulter aufweisen um die Gefahr des Herausrutschens des künstlichen Femurkopfes aus der Pfanne, also die Gefahr der Luxation zu verringern. Solche Gelenkpfannen sind z. B. bekannt als "Poly-Dial inserts" der Joint Medical Products Corporation, Anzeige in "The Journal of Bone and Joint Surgery 68-A, April 1986, S. 91", oder von Polyaethyleneinsätzen der Waldemar Link GmbH & Co., Anzeige in "The Journal of Bone and Joint Surgery, British Volume 68-B, May 1986, nach Seite ii".

Um auch bei ungünstigen Voraussetzungen auf der Becken-Seite, z.B. bei Knochen-Defekten, eine Pfannenprothese einbauen zu können, muss eine grössere Auflagefläche zwischen Metallring und Becken geschaffen werden. Zu diesem Zweck ist aus der deutschen Patentschrift 2314708 eine Metallschale mit einem Auflageflansch bekannt, die mit Knochenzement im Acetabulum verankert wird und in welche, ebenfalls mit Hilfe von Knochenzement, die Polyaethylenpfanne eingefügt wird.

Um trotz verringerter oder geschwächter Knochensubstanz eine zuverlässige Kraftüberleitung von der Prothese auf das Becken zu erreichen, werden Pfannendachschalen aus Metall nach M.E. Müller oder Pfannenstützschalen nach H.B. Burch / R. Schneider verwendet, wie sie z.B. aus R. Schneider : "Die Totalprothese der Hüfte", Bern, Verlag Hans Huber 1982, S. 30 ff" bekannt sind. Die Pfannendachschale nach M.E. Müller und die Pfannenstützschale nach H.B. Burch / R. Schneider sind auch von der Eintragung als Schweizerisches Muster und Modell Nr. 110.826 bzw. 110.827 bekannt.

Diese Pfannendachschalen und Pfannenstützschalen haben wegen ihrer zahlreichen Löcher für die Befestigung mit Schrauben ein siebartiges Aussehen und weisen auf einem Teil ihres Umfanges Auskragungen auf, wodurch eine grossflächige Kraftüberleitung von der Pfanne auf das tragende Knochengewebe bewirkt wird.

Diese Pfannendachschalen oder Pfannenstützschalen werden primär mit Schrauben im Knochen verankert. Die Polyaethylen-Pfanne wird dann mit Hilfe von Knochenzement in der Metallschale befestigt. Dabei dringt der Knochenzement auch durch die Löcher der Metallschale und trägt so zur Befestigung der Metallschale im Knochen bei.

Zur Befestigung des Acetabularteils einer Hüftprothese im Bekken ohne Verwendung von Knochenzement hat P. Schuster in der schweizerischen Patentanmeldung Nr. 3252/84-9 eine Metallschale mit einem im wesentlichen äquatorparallelen Polbereich und strahlenförmig über den Umfang verteilten messerartigen Schneidkanten vorgeschlagen, deren Aussenumriss die Form eines in der Aequatorebene geteilten Achtecks hat, wobei die Möglichkeit der Primärfixation im Beckenknochen durch Schrauben vorgesehen ist.

Der Erfindung liegt die Aufgabe zugrunde, ein Acetabularteil einer Hüftprothese zu schaffen, das aus einer metallenen Schale zur Verankerung im Beckenknochen ohne die Verwendung von Knochenzement und einem in diese Metallschale ebenfalls ohne Zement eingefügten Pfanneneinsatz aus einem geeigneten Material wie Polyaethylen, Metall oder Keramik besteht, wobei ein Kippen und Verdrehen der metallenen Verankerungsschale im Beckenknochen durch konstruktive Ausgestaltung verhindert werden soll.

Die Erfindung löst die gestellte Aufgabe mit dem Acetabularteil, welches die Merkmale des Anspruchs 1 aufweist. Die Gestaltung und die Vorteile der Erfindung werden anhand der Zeichnung erläutert. In der Zeichnung bedeutet :

Fig. 1 eine schematische Darstellung des vollständigen künstlichen Hüftgelenks.

Fig. 3 einen Querschnitt durch die erfindungsgemässe Verankerungsschale und den Pfanneneinsatz in einer für Pfanneneinsätze aus Kunststoff bevorzugten Ausführung.

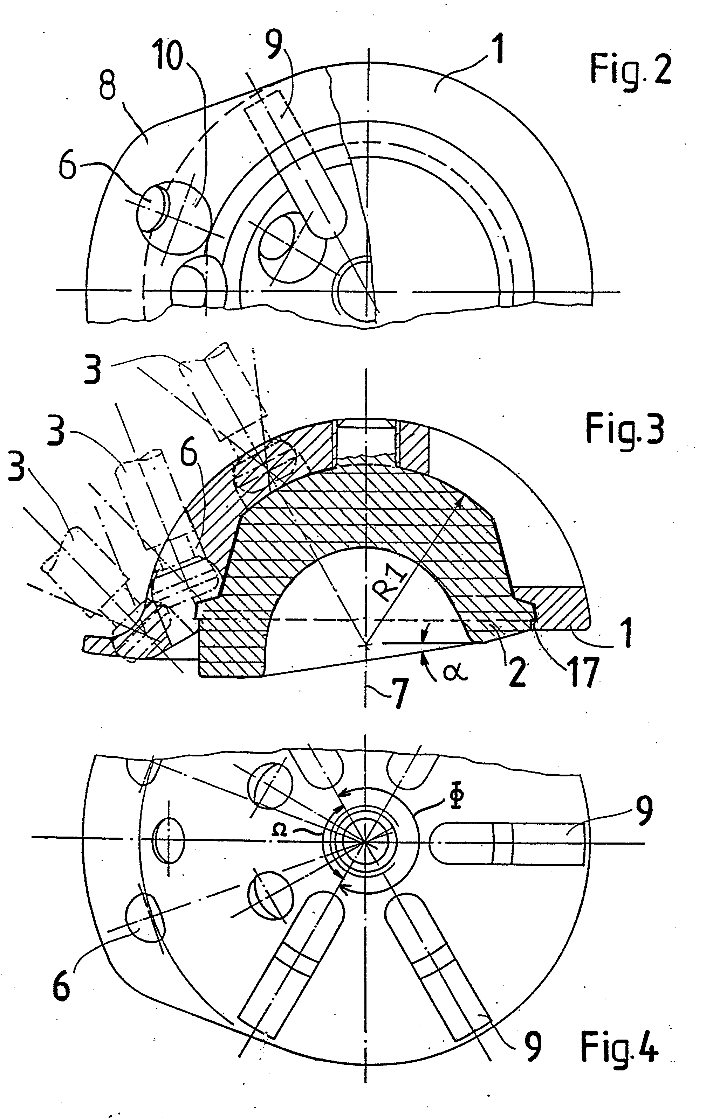

Fig. 2 und 4 Draufsichten auf die Verankerungsschale in einer für Pfanneneinsätze aus Kunststoff bevorzugten Ausführung.

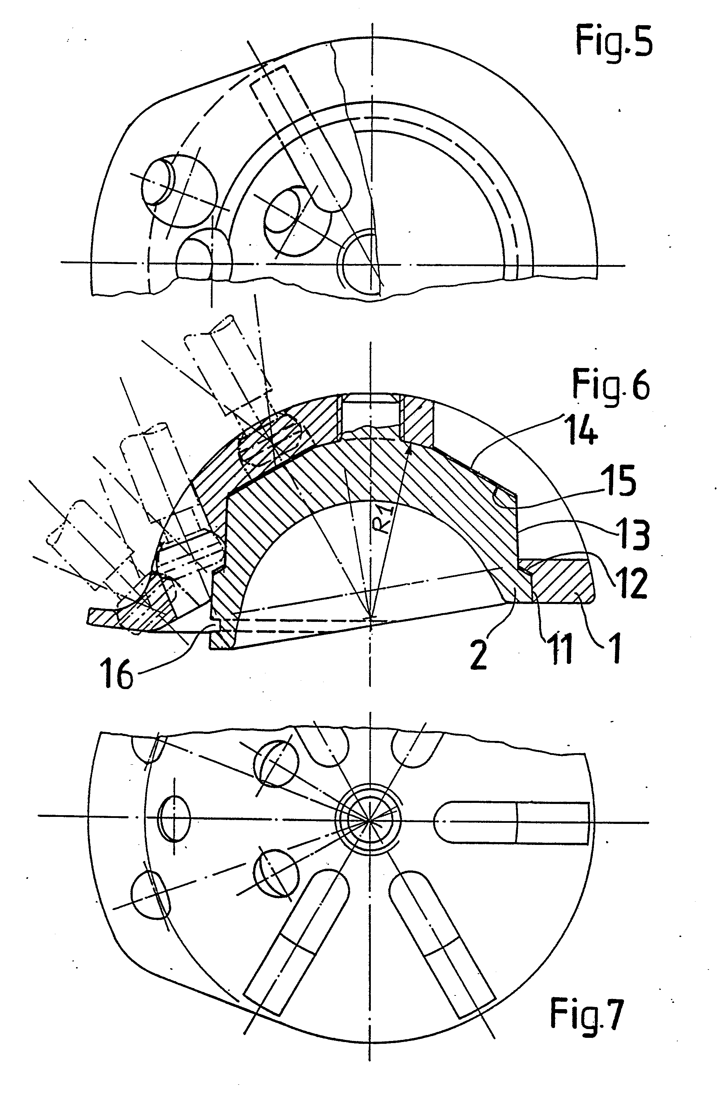

Fig. 6 einen Querschnitt durch die erfindungsgemässe Verankerungsschale und den Pfanneneinsatz in einer für Pfanneneinsätze aus Metall bevorzugten Ausführung.

Fig. 5 und 7 die zu Fig. 6 gehörenden Draufsichten.

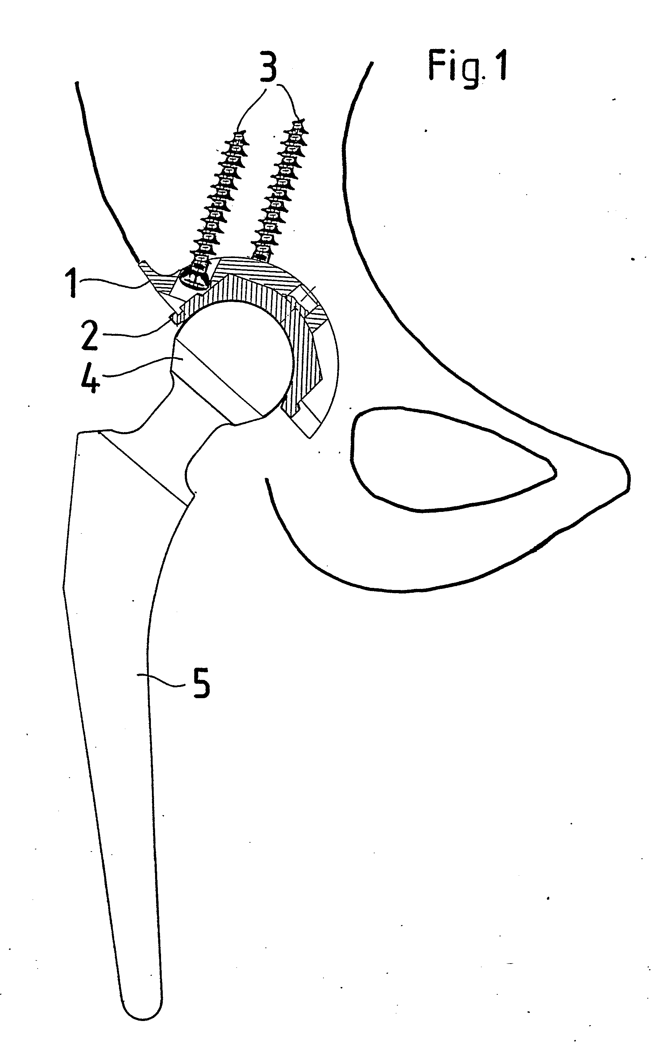

Fig. 1 ist die Frontalansicht der rechten Seite des menschlichen Beckens mit der Verankerungsschale 1, der Pfanne 2 aus Kunststoff oder einem anderen geeigneten Material (z.B. Metall, keramikbeschichtetes Metall, Keramik oder Verbundwerkstoff). Verankerungsschrauben 3, dem künstlichen Femurkopf 4 und dem Prothesenschaft 5.

Fig. 3 zeigt einen Querschnitt durch eine bevorzugte Ausführung der Verankerungsschale 1 mit Bohrungen 6 für Verankerungsschrauben 3, der Pfanne 2 und der Rotationsachse 7 bezogen auf den Konus.

Fig. 2 und 4 zeigen Draufsichten auf eine bevorzugte Ausführungsform der Verankerungsschale 1 mit der Auskragung 8 sowie den Schlitzen 9 und den Bohrungen 6 für die Verankerungsschrauben 3.

Die Verankerungsschale 1 wird bevorzugt aus Rein-Titan hergestellt. Die erfindungsgemässe Konstruktion eignet sich für Pfanneneinsätze 2 aus verschiedenen Materialien wie Polyaethylen, Keramik oder Metall. Einzelne Konstruktionsmerkmale sind jedoch verschieden, je nach dem, ob ein Pfanneneinsatz 2 aus Kunststoff oder einem Werkstoff mit grossem Elastizitätsmodul wie Metall oder Keramik verwendet wird.

Die wesentlichen Konstruktionsmerkmale werden anhand der Fig. 2, Fig. 3, Fig. 4 beschrieben, in denen die für einen Pfanneneinsatz 2 aus Polyaethylen bevorzugte Ausführung dargestellt ist.

Der Wulst 17 des Pfanneneinsatzes aus Kunststoff schnappt in die entsprechende Nut der Verankerungsschale 1 ein, um ein Herausfallen des Pfanneneinsatzes 2 während der Operation bis zum Reponieren des Femurkugelkopfes zu verhindern. Der Femurkugelkopf drückt dann den Pfanneneinsatz 2 gegen die sphärische Fläche mit Radius R 1 und die daran anschliessende Konusfläche.

Das unerwünschte Verdrehen des Pfanneneinsatzes 2 gegen die Verankerungsschale 1 kann dadurch zuverlässig verhindert werden, dass der Pfanneneinsatz 2 mit in Fig. 3 nicht gezeichneten Erhebungen versehen wird, die in die Schlitze 9 eingreifen.

Zur Verringerung der Luxationsgefahr ist der Rand des Pfanneneinsatzes 2 so gestaltet, dass er nicht auf einer Ebene senkrecht zur Rotationsachse 7 des Konus liegt, sondern auf einer um den Winkel $\alpha$ von 90° abweichenden Ebene. Dabei wird $\alpha$ = 10° bevorzugt.

Eine weitere Verbesserung des erfindungsgemässen Acetabularteils besteht in der konstruktiven Gestaltung der Bohrungen 6 für die Verankerungsschrauben 3 in der Weise, dass der Chirurg die in der orthopädischen Chirurgie standardisierten Schrauben 3 unter dem biomechanisch optimalen Winkel, der bis zu 20° oder 30° von der Achse der Bohrung abweicht, eindrehen kann, ohne dass der Schraubenkopf das Einsetzen und Verklemmen des Pfanneneinsatzes 2 behindert, und ohne dass die Wandstärke der Verankerungsschale 1 unerwünscht gross gemacht werden muss.

Das in den Fig. 5, Fig. 6, Fig. 7 dargestellte Acetabularteil, das eine bevorzugte Ausführung für einen Pfanneneinsatz 2 aus Metall zeigt, weist auf der konkaven Innenseite der Verankerungsschale 1 eine zusätzliche Konusfläche 14 und auf der konvexen Aussenseite des Pfanneneinsatzes 2 eine zusätzliche Konusfläche 15 auf, und nur polnahe Teile der Verankerungsschale 1 und des Pfanneneinsatzes 2 haben sphärische Form.

Der Pfanneneinsatz 2 aus Metall verklemmt sich bei Belastung rotationssicher an der Konusfläche 13.

Das Implantieren des erfindungsgemässen Acetabularteils der Hüftprothese erfolgt in folgender Weise : Nach dem Ausfräsen des Acetabulum wird die Verankerungsschale 1 in der zur Kraftüberleitung günstigsten Position mit Hilfe von drei bis fünf Schrauben 3 befestigt.

Die Schlitze 9 erlauben die Kontrolle des guten Kontakts der Verankerungsschale 1 mit dem Knochen.

Durch die Schlitze 9 hindurch kann aus dem resezierten Femurkopf gewonnenes Knochenmaterial oder anderes Knochenmaterial in den unter Umständen zwischen dem ausgefrästen Acetabulum und der Verankerungsschale 1 verbliebenen Raum eingebracht werden.

Dann wird der Pfanneneinsatz 2 in der Weise in die Verankerungsschale 1 eingesetzt, dass der Vektor der resultierenden Kraft der vom Femurkopf auf die Pfanne ausgeübten Kraft annähernd in der Schnittebene liegt, welche durch die Rotationsachse 7 und den höchsten Punkt des Randes des Pfanneneinsatzes 2, gelegt werden kann.

Der nach der Implantation des Acetabularteils in die Schlitze 9 der Verankerungsschale 1 hineinwachsende Knochen unterstützt die stabile Verankerung und erhöht insbesondere die Sicherheit gegen die unerwünschte Rotation der Verankerungsschale 1.

Die Schlitze 9 sind wie in Figur 4 dargestellt längs Meridianen in einem Winkelbereich Φ von 240° geographischer Länge der Kugelkalotte mit gleichem Winkelabstand, angeordnet sind. Im übrigen schlitzfreien Winkelbereich Ω = 360° - Φ geographischer Länge der Kugelkalotte sind die Bohrungen 6 angeordnet.

Die wesentlichen Vorteile der kombinierten Anordnung von Schlitzen 9 und Bohrungen 6 gemäss der Erfindung liegen darin, dass unmittelbar nach der Implantation des Acetabularteils eine genügende Primärfixation erreicht wird, während durch die Möglichkeit der Einfügung von Spongiosamaterial in den Bereich der Schlitze 9 in erster Linie eine Rotationssicherung erreicht wird.

## Ansprüche

1. Acetabularteil für eine Hüfttotalprothese, bestehend aus einer metallischen Verankerungsschale (1), deren dem Beckenknochen zugewandte konvexe Fläche im wesentlichen die Form einer Kugelkalotte hat, mit einem in die Verankerungsschale (1) einsetzbaren Pfanneneinsatz (2), dessen äussere konvexe Form so auf die innere konkave Form der Verankerungsschale (1) abgestimmt ist, dass sie ohne Verwendung von Knochenzement in der Verankerungsschale (1) verdrehsicher fixiert werden kann und dessen innere konkave Form in an sich bekannter Weise die reibungsarme und ungestörte Bewegung eines künstlichen Femurkugelkopfes ermöglicht, dadurch gekennzeichnet, dass die Verankerungsschale (1) mehrere längs Meridianen der Kugelkalotte angeordnete durchgehende Schlitze (9) und mehrere zwischen der Aequatorlinie und dem Pol der Kugelkalotte liegende Bohrungen (6) zur zementlosen Primärverankerung im Beckenknochen mittels Verankerungsschrauben (3) aufweist.

2. Acetabularteil nach Anspruch 1, dadurch gekennzeichnet, dass die Schlitze (9) in der Verankerungsschale (1) mindestens 3 mm und höchstens 7 mm breit sind.

3. Acetabularteil nach Anspruch 1 oder 2, dadurch gekennzeichnet, dass die Schlitze (9) in der Verankerungsschale (1) überall gleich breit sind.

4. Acetabularteil nach einem der Ansprüche 1 bis 3, dadurch gekenzeichnet, dass die Schlitze (9) an mindestens einem Ende die Form eines Halbkreises aufweisen.

5. Acetabularteil nach einem der Ansprüche 1 bis 4, dadurch gekennzeichnet, dass die Schlitze (9) längs Meridianen in einem Winkelbereich Φ von mindestens 210° und höchstens 270° geographischer Länge der Kugelkalotte, vorzugsweise mit gleichem Winkelabstand, angeordnet sind.

6. Acetabularteil nach einem der Ansprüche 1 bis 5, dadurch gekennzeichnet, dass der polnahe Anfang der Schlitze (9) auf einem Breitenkreis der Kugelkalotte mit mindestens 12 mm Durchmesser liegt und die Schlitze (9) bis höchstens 5 mm an den Rand der Verankerungsschale (1) heranreichen.

7. Acetabularteil nach einem der Ansprüche 1 bis 6, dadurch gekennzeichnet, dass am Pfanneneinsatz (2) Erhebungen vorgesehen sind, die in die Schlitze (9) der Verankerungsschale (1) eingreifen.

8. Acetabularteil nach einem der Ansprüche 1 bis 7, dadurch gekennzeichnet, dass die dem Beckenknochen zugewandte konvexe Fläche der Verankerungsschale (1) im wesentlichen die Form einer Kugelkalotte hat und der Rand der Verankerungsschale (1) auf minimal 70° und maximal 140° seines Umfanges eine Auskragung (8) zur Vergrösserung der Berührungsfläche zwischen Verankerungsschale (1) und Beckenknochen aufweist.

9. Acetabularteil nach Anspruch 5, dadurch gekennzeichnet, dass die Bohrungen (6) nur im schlitzfreien Winkelbereich Ω = 360° - Φ geographischer Länge der Kugelkalotte angeordnet sind.

10. Acetabularteil nach einem der Ansprüche 1 bis 9, dadurch gekennzeichnet, dass die Bohrungen (6) auf der konkaven Seite der Verankerungsschale (1) einen Raum (10) aufweisen, der die Köpfe der Verankerungsschrauben (3) vollständig in der Weise aufnimmt, dass die Schraubenköpfe nicht über die konkave Innenfläche der Verankerungsschale (1) nach innen herausragen.

11. Acetabularteil nach einem der Ansprüche 1 bis 10, dadurch gekennzeichnet, dass der Raum (10) für die Schraubenköpfe derart ausgebildet ist, dass die Verankerungsschrauben (3) mit einer dem Raum (10) angepassten Schraubenkopfpartie unter einem von der Mittelachse der Bohrung (6) um mindestens 20°, vorzugsweise mindestens 30° abweichenden Winkel einschraubbar sind.

12. Acetabularteil nach einem der Ansprüche 1 bis 11, dadurch gekennzeichnet, dass der Raum (10) für die Schraubenköpfe die Form des Teils eines Ellipsoides aufweist.

13. Acetabularteil nach einem der Ansprüche 1 bis 12, dadurch gekennzeichnet, dass am Rand des Pfanneneinsatzes (2) ein Wulst (17) und in der Verankerungsschale (1) eine entsprechende Nut vorgesehen ist, in welcher sich der Wulst (17) verklemmt.

14. Acetabularteil nach einem der Ansprüche 8 bis 13, dadurch gekennzeichnet, dass die dem Beckenknochen zugewandte konvexe Fläche der Verankerungsschale (1) von der Fläche einer Kugelkalotte abweicht, derart, dass zwischen der Kugelfläche und Auskragung (8) des Randes ein stetiger Uebergang ohne Kerben und Kanten besteht.

15. Acetabularteil nach einem der Ansprüche 8 bis 14, dadurch gekennzeichnet, dass die dem Beckenknochen zugewandte konvexe Fläche der Verankerungsschale (1) von der Fläche einer Kugelkalotte abweicht, derart, dass am Pol der Kugelkalotte abgeflacht ist.

16. Acetabularteil nach einem der Ansprüche 1 bis 15, dadurch gekennzeichnet, dass die konkave Innenfläche der Verankerungsschale (1) im Boden einer mit Schlitzen versehenen Kugelkalotte entspricht und zum Schalenrand hin in einen Konus übergeht.

17. Acetabularteil nach einem der Ansprüche 1 bis 15, dadurch gekennzeichnet, dass die konkave Innenfläche der Verankerungsschale 1 eine Reihe von Konusflächen (11), (12), (13), (14) aufweist und die Abmessungen der Verankerungsschale (1) und des Pfanneneinsatzes (2) so gewählt sind, dass sich der Pfanneneinsatz (2) an den Konusflächen (13) in der Verankerungsschale (1) verklemmt.

18. Acetabularteil nach Anspruch 17, dadurch gekennzeichnet, dass ein Teil des Umfangs der Konusfläche (11) des Pfanneneinsatzes (2) eine Nut (16) aufweist.

19. Acetabularteil nach einem der Ansprüche 1 bis 17, dadurch gekennzeichnet, dass die Rotationsachse (7) des Konus der dem Beckenknochen abgewandten konkaven Fläche der Verankerungsschale (1) identisch ist mit der Achse der Kugelkalotte der Aussenfläche.

20. Acetabularteil nach einem der Ansprüche 1 bis 19, dadurch gekennzeichnet, dass die dem Becken zugewandte konvexe Form des rotationssymmetrischen Teils des Pfanneneinsatzes (2) ein Abbild des rotationssymmetrischen Teils der konkaven Innenform der Verankerungsschale (1), jedoch ohne Berücksichtigung der Schlitze (9) ist, und dass die Abmessungen der Verankerungsschale (1) und des Pfanneneinsatzes (2) so gewählt sind, dass sich der Pfanneneinsatz (2) verdrehsicher in der Verankerungsschale (1) verklemmbar ist.

21. Acetabularteil nach einem der Ansprüche 1 bis 20, dadurch gekennzeichnet, dass die Ebene des Randes des Pfanneneinsatzes (2) mit der Rotationsachse (7) des rotationssymmetrischen Teiles des Pfanneneinsatzes (2) einen von 90° abweichenden Winkel bildet.

22. Acetabularteil nach Anspruch 21, dadurch gekennzeichnet, dass der von 90° abweichende Winkel grösser als 80° und kleiner als 90° ist.

23. Acetabularteil nach einem der Ansprüche 1 bis 22, dadurch gekennzeichnet, dass eine Reihe von Pfanneneinsätzen (2) mit an sich bekannter Innenform als Gegenlager für künstliche Femurköpfe (4) mit verschiedenen Kugelkopfdurchmessern, jedoch mit gleichen Verankerungsschalen (1), vorgesehen sind.

Fig. 1

0 265 712

Fig. 2

Fig. 3

Fig. 4

Fig.5

Fig.6

Fig.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | DE-A-3 310 944 (F. ERLER)<br>* Figuren 1-3,8; Zusammenfassung * | 1,3,10 | A 61 F 2/34 |
| Y | DE-A-2 822 585 (WALDEMAR LINK)<br>* Ansprüche 1-4; Figuren 1-3 * | 1,3,10 | |
| A | US-A-3 903 549 (W.M. DEYERLE)<br>* Figur 1; Zusammenfassung * | 1,7 | |
| A | US-A-3 608 096 (WALDEMAR LINK)<br>* Figur 2 * | 5 | |
| A | EP-A-0 091 315 (N.R.D.C.)<br>* Figur 6 * | 8,14 | |

RECHERCHIERTE SACHGEBIETE (Int. Cl.4)

A 61 F

Der vorliegende Recherchenbericht wurde für alle Patentansprüche erstellt

| Recherchenort | Abschlußdatum der Recherche | Prüfer |
|---|---|---|
| DEN HAAG | 21-01-1988 | NEILL M.C. |

EPO FORM 1503 03.82 (P0403)